(19) Europäisches Patentamt
European Patent Office
Office européen des brevets

(11) Publication number: **0 200 565**
**A2**

# (12) EUROPEAN PATENT APPLICATION

(21) Application number: **86303372.6**

(22) Date of filing: **02.05.86**

(51) Int. Cl.[4]: **C 12 N 9/08**
C 12 N 1/14
//(C12N1/14, C12R1:645)

(30) Priority: **02.05.85 JP 95837/85**

(43) Date of publication of application:
**05.11.86 Bulletin 86/45**

(84) Designated Contracting States:
**CH DE FR GB IT LI**

(71) Applicant: **NISSIN FOOD PRODUCTS CO., LTD.**
**4-1-1 Nishinakajima Yodogawa-ku**
**Osaka 532(JP)**

(72) Inventor: **Terada, Masaki**
**6-308 Zezekoendanchi**
**6 Honmaru-cho Ohtsu 520(JP)**

(72) Inventor: **Takatsu, Mitsumune**
**3-5-16 Kusatsu**
**Kusatsu 525(JP)**

(72) Inventor: **Minami, Junichi**
**2-9-19 Kunokidai**
**Tondabayashi 584(JP)**

(74) Representative: **Hayward, Denis Edward Peter et al,**
**Lloyd Wise, Tregear & Co. Norman House 105-109 Strand**
**London WC2R 0AE(GB)**

(54) **Method for production of peroxidase.**

(57) Disclosed by the present invention are methods for the production of peroxidase enzyme comprising the culturing of selected species and strains of the genus *Coprinus* and isolating peroxidase enzyme from said organism and accumulated in the medium.

EP 0 200 565 A2

"METHOD FOR PRODUCTION OF PEROXIDASE"

The present invention relates generally to methods for producing peroxidase and particularly to methods utilizing microorganisms for its production.

Peroxidase is an enzyme which oxidizes a variety of chemical compounds (including chromogens) in the presence of hydrogen peroxide. The enzyme is used as a diagnostic reagent and as a catalyst for chemical synthesis. It is desirable that peroxidase materials obtained for use as diagnostic reagents have a wide range of specificity to chromogens. It is particularly desirable to obtain peroxidase which develops color in the presence of a widely used chromogen indicator solution comprising 4-aminoantipyrine and phenol. Illustratively, the degree of sterilization of cow's milk, which naturally contains peroxidase, can be determined by measurement of the amount of active peroxidase present in cow's milk before sterilization and the amount present after sterilization. While many other food products which are subjected to heat treatment do not naturally contain peroxidase, peroxidase may be added to the product in order to monitor the heat treatment by testing for peroxidase activity before and after treatment. It is essential, however, that any peroxidase added to a food product be pure and hygienically acceptable.

Peroxidase has also found applications in food processing technology for measurement of the degree of

heat-treatment of various foods. Peroxidase is known to be present in horseradish, sweet potato, leaves of broad bean and wasabi, as well as in leukocytes and cow's milk.

Horseradish is currently the primary industrial source of peroxidase. Unfortunately, the supply of horseradish and the other plants from which the enzyme may be derived varies annually and supplies of peroxidase of animal origin have proved difficult to obtain in bulk. Because of the uncertainty of supply of peroxidase produced by conventional methods, work has been directed toward methods for securing microbial synthesis of peroxidase in quantity. One early attempt to produce peroxidase used *Streptococcus faecalio* [*J. Biol. Chem.*, Vol. 225, 557 (1957)], while recent attempts have used organisms of the genus *Alternalia*, *Cochliobolus*, *Pellicularia*, *Courvularia* [Japanese Patent 83005035], and *Oidiodendron* [Japanese Patent Application No. 179075, 1984]. See also, published European Patent Application 171074 relating to the use of *Arthromyces* species. It has been found, however, that peroxidase of *Streptococcus* origin is not appropriate as a reagent since it is a NADH-peroxidase. Furthermore, the peroxidases produced by many of the microorganisms of the prior art have not been used for food processing, and their safety is not yet established. There thus continues to be a need in the art for an inexpensive, reliable and hygienically acceptable source of peroxidase enzymes.

Of interest to the background of the present invention references generally relating to fungi of the genus *Coprinus* (see, e.g. pages 92-95, American Type Culture Collection, 'Catalogue of Fungi/Yeasts' 16th Edition, 1984) and particularly references relating to prospects for obtaining useful enzymes through culturing various Coprinus species (see, e. g. United

States Patent 3,694,316 (protease), Japanese Patent Application 60156385 (laccase), published European Patent Application 148950 (bilirubin oxidase), Japanese Patent Application 59140880 (lipoprotein lipase), and Japanese Patent 85024710 (cellulase)]. To date, there have been no reports of the use of fungi of the genus Coprinus in production of peroxidase enzymes.

According to the present invention peroxidase enzyme is isolated from fungi of the genus Coprinus. Preferred methods are disclosed for the production of peroxidase comprising the culturing (incubating) of selected species and strains of microorganisms belonging to the genus Coprinus in a suitable growth medium and the isolating peroxidase enzyme accumulated in the medium. Particularly preferred organisms for practice of the invention include Coprinus cinereus (IFO 30116), Coprinus cinereus f. microsporus (IFO 7662) and Coprinus filamentifer (IFO 31058). Fungi of the genus Coprinus have long been used as a source of edible mushrooms. Consequently, peroxidase enzymes isolated from these fungi are expected to be hygienically acceptable when employed to monitor heat treatment of food products.

Other aspects and advantages of the present invention will be apparent to those skilled in the art upon consideration of the following detailed description of illustrative embodiments thereof and wherein:

Figure 1 shows the activity vs. pH curve of peroxidase (Coprinus cinereus IFO 30116) according to the present invention;

Figure 2 shows the pH stability of said peroxidase; and

Figure 3 shows the temperature stability of said peroxidase.

The present invention relates generally to production of peroxidase by fungi of the genus Coprinus. Preferred methods for the production of peroxidase comprise culturing (incubating) of microorganisms of the genus Coprinus capable of producing peroxidase in a suitable culture medium and thereafter isolating peroxidase accumulated in the medium.

Samples of various strains of Coprinus were obtained from the Institute for Fermentation, Osaka, Japan ("IFO") and were cultured in either a potato-sucrose culture medium (comprising 200 grams of potato and 20 grams of sucrose dissolved in one liter of water and adjusted to pH 5-6) or in the Matsutake mushroom culture medium (comprising 5 grams of Ebois and 20 grams of glucose dissolved in one liter of water adjusted to pH 4.5) [described on page 279 of the List of Cultures, 7th edition, published by the IFO]. Ten milliliter samples of medium were then placed in L-shaped test tubes and autoclaved for 20 minutes at 121°C. The sterile medium samples were then inoculated with samples of various strains of Coprinus using a platinum loop. The culture samples were then incubated by shaking at 100 r.p.m. and 25°C for about one week to produce a seed fungus. One hundred milliliters of media was taken separately in a 500 milliliter Sakaguchi's flask and sterilized. This material was then inoculated with 10 milliliters of the seed fungus. The culture was then incubated by shaking at 25°C for three days.

Filtrate was collected from the culture media and assays were conducted to determine the peroxidase

activities of various strains of Coprinus. Substrate solutions were made up comprising 1.5 milliliters (0.0017M) hydrogen peroxide aqueous solution mixed with 1.4 milliliters of a solution comprising 0.0025M 4-aminoantipyrine and 0.17M phenol. Assays were conducted by adding a 0.1 milliliter sample of each enzyme containing media solution to the substrate solution, which was then incubated for 3 to 4 minutes at 25°C. The absorbance at 510 nm was measured continuously by a spectrophotometer and enzyme activity was measured by the rate of increase in absorbance ($\Delta A_{510}$/min). The results of the assay are presented in Table 1.

All of these fungi are known deposited fungi registered on the List of Cultures, 7th edition (1984) published by the IFO and can be obtained from the Institute. The fact that many of these fungi are ordinarily consumed as edible mushrooms, provides support for the proposition that these fungi tend to be safe in comparison with certain of the microorganisms employed to produce peroxidase enzymes in the prior art. While some of the fruitbodies are known to produce unpleasant effects when consumed with alcohol, young fungi are disclosed to pose no such problem.

TABLE 1

| Name of Fungus | | Activity (ΔA/min) |
|---|---|---|
| Coprinus angulatus | IFO 30971 | Trace |
| Coprinus atramentarius | IFO 30626 | Trace |
| Coprinus bilanatus | IFO 30122 | 0.001 |
| Coprinus cinereus | IFO 30116 | 0.082 |
| Coprinus cinereus | IFO 30628 | 0.186 |
| Coprinus cinereus | IFO 31333 | 0.163 |
| Coprinus cinereus f. microsporus | IFO 7662 | 0.057 |
| Coprinus comatus | IFO 30325 | 0.002 |
| Coprinus disseminatus | IFO 7550 | Trace |
| Coprinus echinosporus | IFO 30630 | Trace |
| Coprinus filamentifer | IFO 31058 | 0.026 |
| Coprinus fissolanutus | IFO 30124 | Trace |
| Coprinus lagopides | IFO 30120 | 0.001 |
| Coprinus macrocephalus | IFO 30117 | 0.007 |
| Coprinus neolagopus | IFO 30476 | 0.001 |
| Coprinus ochraceo-velatus | IFO 30121 | Trace |
| Coprinus phlyctidosporus | IFO 30477 | Trace |
| Coprinus pseudolagopus | IFO 30123 | Trace |
| Coprinus radiatus | IFO 30118 | 0.002 |
| Coprinus rhizophorus | IFO 30197 | 0.001 |
| Coprinus sp | IFO 30125 | 0.002 |
| Coprinus sp | IFO 30126 | 0.007 |
| Coprinus strercorarius | IFO 30479 | Trace |

The fungi of the present invention may be cultured in media which contain ordinary nutrients and additives. Suitable carbon sources include saccharides such as glycerin, glucose, galactose, mannose, fructose, maltose, sucrose, raffinose, xylose, starch, thick malt syrup and sorbitol; organic acids and fatty acids such as acetic acid, citric acid, succinic acid, butyric acid and palmitic acid; alcohols such as methyl alcohol, ethyl alcohol and amyl alcohol; and hydrocarbons such as hexane and octane. Suitable nitrogen sources include natural nitrogen sources such as defatted soybean meal, casein, peptose, meat extract, yeast extract, corn steep

liquor and various amino acid mixtures; and inorganic nitrogen sources such as ammonium sulfate, ammonium nitrate, ammonium chloride and urea. Inorganic salts such as phosphate, chloride and sulfate, and vitamins may be added when necessary. The specific proportions of the materials of culture medium may be varied to suit each species or strain of microorganism. The optimum pH and temperature of the medium may also be varied according to the specific fungus, but pH may generally range from 2 to 10, with temperatures ranging from 10 to 40°C. More preferably, pH should be in the range from 4 to 8, and the temperature should range from 15 to 35°C. The microbes may be cultured be either liquid or solid culture techniques with their periods of culture normally running from 2 to 7 days, and 10 to 60 days, respectively.

Peroxidase produced by the microbes of the invention may either be contained within the cells of the fungi or may be secreted outside the cells. Where peroxidase is secreted to the culture medium, the medium itself may be directly filtered. If it is not secreted, and is instead accumulated inside the fungus cells, the cells may be homogenized by standard means and then filtered.

Where the culturing step is carried out in a solid phase, extraction may be accomplished by addition of water or an appropriate salt solution to the solid medium in which the fungus has grown. Separation of the peroxidase enzyme can then be accomplished by ordinary methods such as treatment with ammonium sulfate, dialysis, and precipitation with organic solvents, as well as through use of various chromatographic procedures and electrophoresis, either singly or in combination.

EXAMPLE 1

In this example, peroxidase isolated according to the methods described above from the culture medium of Coprinus cinereus IFO 30116 was assayed to determine its activity. A sample comprising 0.1 ml of the isolated enzyme was added to 1.4 milliliters of a chromogen mixture comprising 0.0025M 4-aminoantipyrine and 0.17M phenol and 1.5 milliliters of 0.2M phosphate-buffered solution (pH 7.0). The mixture was incubated at 25°C, and the light absorbance at 510 nm was measured continuously during the period from 30 seconds to 2 minutes after addition of the enzyme. The activity of this sample of peroxidase was measured by the rate of increase of light absorbance at 510 nm ($\Delta A_{510}$/min) and its activity was defined as one activity unit.

EXAMPLE 2

In this example, peroxidase produced by Coprinus cinereus IFO 30116 was isolated and partially purified. Potato-sucrose (PS) medium was prepared mixing 200 grams of potato, 20 grams of sucrose and sufficient water to make up 1 liter. (see The List of Cultures, 7th edition, p. 279, published by the IFO). Seventy milliliters of this mixture was added to a 500 milliliter Sakaguchi's flask and sterilized at 120°C for 30 minutes. At the same time, approximately 1 square centimeter of Coprinus cinereus IFO 30116 cultured on a slant medium was cut out from the medium and aseptically and mildly crushed. The PS culture medium was then inoculated with the Coprinus cinereus culture and the fungus was incubated in a rotary shaker at 27°C for seven days. The culture medium was strained through a cloth filter and to 60 ml of the resulting filtrate was added 370 grams of ammonium sulfate. The mixture was

left to stand at 4.5°C for one night and was then filtered with celite. The precipitate was collected, dissolved in a small amount of water, and put in a dialysis tube. Dialysis against 2.5 x $10^4$M $FeCl_3$ aqueous solution (pH 7.0) produced 18 milliliters of internal liquid. At this point, the activity of peroxidase was 45.7% of the total activity of the culture filtrate. The internal liquid was then adsorbed by a 18ϕ x 230 mm DEAE cellulose column treated with 0.01M phosphate buffer solution (pH 7.5). The solution was then eluted with a solution comprising 0.1M NaCl in a 0.1M phosphate buffer solution. The eluent was then concentrated by ultrafiltration (5000 molecular weight cutoff), and 10.5 milliliters of crude enzyme solution was obtained. The crude enzyme solution demonstrated a total activity, 15% of that of the culture filtrate.

Many of the enzymatic and chemical properties of the isolated peroxidase enzyme may be characterized as follows. The enzyme acts primarily on hydrogen peroxide, and oxidizes a variety of hydrogen donors in the presence of hydrogen peroxide. Its action is shown by the following reaction formula:

$$H_2O_2 + AH_2 \rightarrow 2H_2O + A$$

where $AH_2$ is a hydrogen donor, and A is the oxidized hydrogen donor.

The enzyme was assayed for activity with respect to a variety of hydrogen donors. The results shown in Table 2 demonstrate that the enzyme acts particularly effectively upon chromogens with amino groups.

TABLE 2

| Hydrogen donor | Strength of Action |
| --- | --- |
| Phenol | 100 (%) |
| Hydroquinone | 57 |
| Resorcinol | 0 |
| Catechol | 460 |
| o-dianisidine | 5400 |
| Guaiacol | 200 |
| α-naphthol | 1200 |
| δ-hydroquinon | 57 |
| o-tolidine | 5400 |

Values shown in Table 2 are relative ones with that of phenol set at 100%.

The activity of the enzyme was also measured at various pHs with the results shown in Figure 1. The enzyme acted in a range from pH 3 to pH 11, with the optimal range being from pH 6 to pH 9.

The stability of the peroxidase enzyme was determined at various pHs with the results shown in Figure 2. Samples of the enzyme were incubated at 25°C and at various pHs for 22 hours. The samples were then tested for activity at pH 7. As shown in Figure 2, the peroxidase enzyme was stable in the range from pH 4 to pH 9 with peak stability at pH 8.

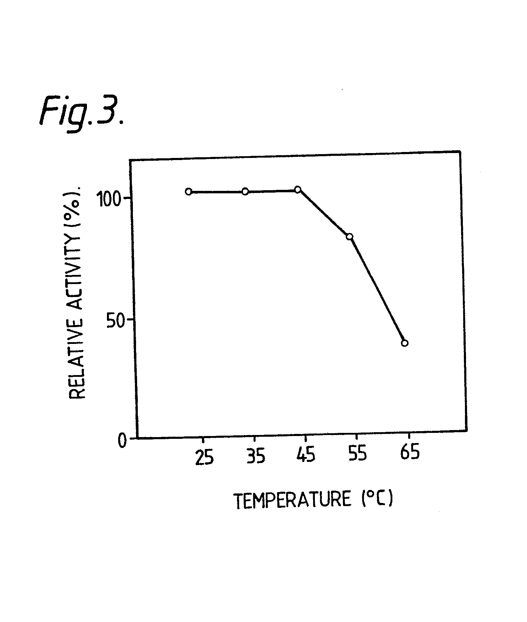

The temperature stability of the enzyme was tested by heating the enzyme in 0.1M phosphate-buffered (pH 8.0) solution at various temperatures for 10 minutes. Activity was unaffected by heating at 45°C or lower. After heating at 55°C for 10 minutes, greater than 50% of its reactivity remained (see Figure 3).

EXAMPLE 3

In this example a preferred liquid culturing embodiment of the present invention was performed wherein a PS medium comprising 50 gms of sucrose and 500

grams of potato infusion to which sufficient water was added to produce 2.5 liters of the medium (pH 5-6) was put in a jar fermenter with a capacity of 5 liters and sterilized at 120°C for 60 minutes. One hundred milliliters of a seed fungus, Coprinus Cinereus IFO 30116 precultured on the same medium was inoculated on the PS medium so prepared. The culture was then incubated at 28°C while undergoing stirring at 350 to 450 rpm and aeration at the rate of 1 liter/minute. Incubation continued for 3 days, at which time the culture medium yielded peroxidase material with activity of 283 units per ml.

EXAMPLE 4

In this example a preferred solid culturing embodiment of the present invention was performed wherein a solid medium was prepared by mixing 2.5 grams of sucrose and 100 grams of cellulose powder with 125 milliliters of infusion obtained from 200 grams of potato treated with 1 liter of boiling water. The medium was sterilized at 120°C for 20 minutes. Approximately 10 platinum loops of a crushed test tube slant culture of Coprinus cinereus IFO 30114 were added to the medium and mixed well with it. The medium was then formed into a layer of about 0.5 cm thick, and was incubated at 25°C for 14 days in a static culture. Five hundred milliliters of water was then added to the culture, and the culture was left to stand at room temperature for 60 minutes. Peroxidase obtained from the filtrate of the culture had activity measured at 11 units per ml.

According to the present invention, peroxidase can be produced by culturing a peroxidase producing microorganism belonging to the genus Coprinus and collecting the peroxidase produced either directly from the

cells or from the culture medium. Because peroxidase obtained according to the present invention acts well particularly on amino chromogens such as 4-aminoantipyrine, o-dianisidine and o-tolidine, it may be used as diagnostic reagent. Because many of the microorganisms are edible, peroxidases produced by these microorganisms represent fewer food safety concerns and should have a wider range of applications especially in monitoring heat sterilization of food products.

What is claimed is:

1. A method for producing peroxidase comprising culturing a microorganism belonging to genus Coprinus and being capable of producing peroxidase in a suitable culture medium and isolating peroxidase from said microorganism.

2. A method for producing peroxidase comprising culturing a microorganism belonging to genus Coprinus and being capable of producing peroxidase in a suitable culture medium and isolating peroxidase from said culture medium.

3. A method according to claim 1 wherein the microorganism is selected from the group consisting of: Coprinus cinereus IFO 30114, Coprinus cinereus IFO 30116, Coprinus cinereus IFO 31333, Coprinus cinereus IFO 30628, Coprinus cinereus f. microsporus IFO 7662 and Coprinus filamentifer IFO 31058.

4. A method according to claim 2 wherein the microorganism is selected from the group consisting of: Coprinus cinereus IFO 30114, Coprinus cinereus IFO 30116, Coprinus cinereus IFO 31333, Coprinus cinereus IFO 30628, Coprinus cinereus f. microsporus IFO 7662 and Coprinus filamentifer IFO 31058.

Fig.1.

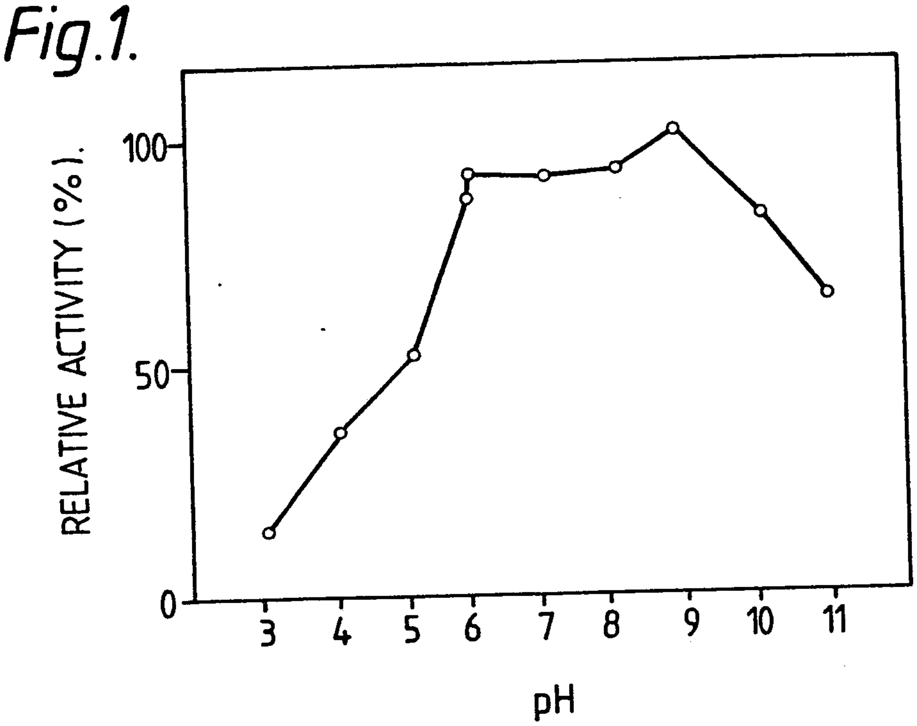

RELATIVE ACTIVITY (%).
100
50
0
3
4
5
6
7
8
9
10
11
pH


Fig.2.

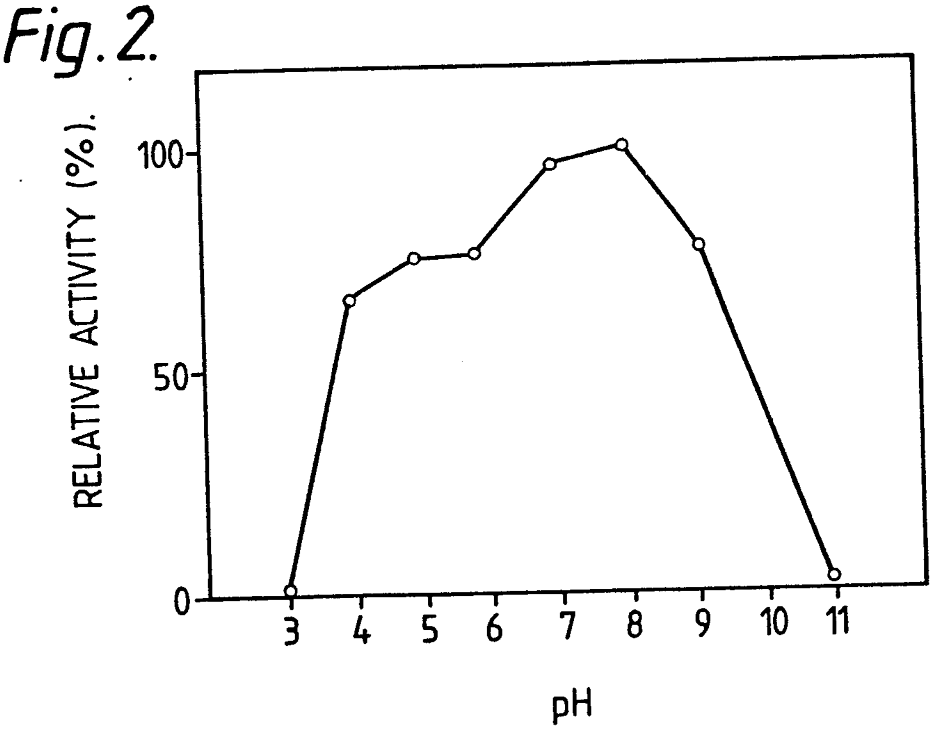

RELATIVE ACTIVITY (%).
100
50
0
3
4
5
6
7
8
9
10
11
pH

Fig.3.